# NEW EUROPEAN PATENT SPECIFICATION

(11) **EP 0 644 767 B3**
(45) Date of publication of this specification: **08.04.2009**
(45) Mention of the grant of the patent: 10.09.1997
(21) Application number: 93913325.2
(22) Date of filing: 09.06.1993
(51) Int. Cl.: A61K 33/14, A61K 33/42, A61K 31/70, A61K 31/19

(54) **VETERINARY REHYDRATION PRODUCT**
VETERINAERPRODUKT ZUR REHYDRATION
PRODUIT VETERINAIRE DE REHYDRATATION

(30) Priority: 16.06.1992 GB 9212737
(43) Date of publication of application: 29.03.1995
(73) Proprietor: NORBROOK LABORATORIES LIMITED, Newry, County Down BT35 6JP (GB)
(72) Inventor: HOLMES, Drew, Bryansford, Newcastle BT33 0FL (GB); PATTERSON, Alan, Belfast BT4 2NQ (GB)
(74) Representative: Hayes, Adrian Chetwynd
(86) International application number: PCT/GB1993/001220
(87) International publication number: WO 1993/025214

(56) References cited:
- EP-A2- 0 459 108
- WO-A1-85/01657
- WO-A1-87/06832
- BE-A2- 897 402

## Description

This invention relates to veterinary products for the treatment of livestock suffering from disorders which may arise from nutritional, bacterial, viral or protozoal causes and lead to fluid depletion, acidosis, and imbalances or loss of essential electrolytes. These problems typically arise in immature animals such as calves and lambs.

The intestinal losses of water and electrolytes, including bicarbonate, are major factors in the pathogenesis of diarrhoea in calves. Calf scour continues to represent a major financial loss to the farmer and also a great deal of concern to the veterinary profession. Thus, this invention is primarily intended to be applied in rehabilitating calves suffering from scour and description of the invention herein will be related thereto. However, it will be appreciated that other animals may also suffer from abnormal conditions leading to similar dehydration, acidosis and electrolyte loss. Thus it could be used for promoting recovery of competition horses severely stressed from fluid loss after a vigorous event.

The causes of dehydration have been well researched and although many interacting factors have to be considered it is observed that severe dehydration includes reduced fluid absorption as well as increased excretion and is accompanied by loss of sodium and other electrolytes from the extracellular fluids. Acidosis, where blood pH drops from about 7.34 - 7.40 to a lower range of perhaps 6.85 - 7.15, is mainly attributable to increases in loss of bicarbonate ion, increases in lactic acid which dissociates to the lactate and hydrogen ions, and additional factors such as increases in other organic acid levels produced by infecting bacteria.

Losses of sodium and bicarbonate ions are considered to be the most serious aspects of diarrhoea but losses of potassium and calcium are also observed.

There are already many veterinary products currently available which have varying degrees of efficacy with respect to replacement of electrolytes, correction of acidosis and provision of an energy source. The Applicant's own Life Aid P (trade mark) is one such rehydration product, whilst another is Lectade Plus (trade mark), of SmithKline Beecham Animal Health.

Whereas rehydration products may be administered intravenously, orally, or subcutaneously, the oral route is much preferred unless dehydration has already progressed to a serious level (perhaps 6 to 10% or above) when a combination of parenteral and oral administration is employed.

An object of this invention is to provide an improved veterinary product providing for effective rehydration whilst countering metabolic acidosis more effectively than was previously possible.

Accordingly, this invention provides a composition to be made up in water at point of use as an oral rehydration formulation, which composition comprises an intimate mixture of an energy source, electrolytes, and bicarbonate precursors, which precursors are provided as a plurality of physiologically acceptable carboxylic acid anions with corresponding physiologically acceptable cations including sodium, wherein the bicarbonate precursors are selected from alkali metal salts of a tricarboxylic acid and two other carboxylic acids, the yield of bicarbonate being from about 30 millimole/litre (mmol/l) to about 100 mmol/l of final formulation, and the concentration of sodium being in excess of 80 mmol/l to about 133 mmol/l of final formulation.

The invention also provides an oral rehydration formulation comprising an aqueous solution of an intimate mixture of an energy source, electrolytes, and bicarbonate precursors, which precursors are provided as a plurality of physiologically acceptable carboxylic acid anions with corresponding physiologically acceptable cations including sodium, the yield of bicarbonate being from about 30 millimole/litre (mmol/l) to about 100 mmol/l of final formulation, and the concentration of sodium being in excess of 80 mmol/l to about 133 mmol/l of final formulation. The composition preferably also contains potassium chloride and may contain potassium dihydrogen orthophosphate to obtain an ionically balanced product.

Preferably, the composition contains differing bicarbonate precursors to increase the probability of absorption and achieves a yield of about 50 to 100 mmol/l bicarbonate. The group of precursors includes alkali metal especially sodium, salts of a tricarboxylic acid and two other carboxylic acids. Propionate (propanoate), acetate and citrate are the preferred bicarbonate precursors being converted to this anion in vivo to yield the desired concentration. Metabolism of these precursors also contributes to the energy yield of the present product.

The composition of this invention also contains a significantly greater level of energy source than prior art compositions preferably in excess of 160 mmol/l, and this is preferably as glucose to facilitate sodium uptake. A slight hypertonicity has been shown to be tolerable (osmality 391.7 mmol/l in comparison with normal calf plasma at about 300 mmol/l) and this may arise if final glucose concentration is in excess of about 3% but much greater concentrations are to be avoided.

The composition is preferably provided in particulate form e.g. as a substantially dry powder or granules but may alternatively be provided as a liquid concentrate for dilution when required for administration.

In the form of an intimate mixture of dry (typically not more than about 1.8% w/w water content) components of this invention (which may include a desiccant), the formulation is storage stable for extended periods.

This new product may be produced for distribution as a substantially dry powder composition, or as a liquid concentrate for make up to a dosage formulation at point of use. Whereas prior formulations (including Life Aid P^{™}) have typically been distributed as two part powder compositions for subsequent mixing and make up with water for use, one unique aspect of this invention lies in the composition being packaged as a single mix in a dosage quantity in a container such as a sachet, or capsule for example. This "one dosage per animal per sachet" packaging greatly simplifies administration. Thus the product may be ideally packaged in sachets connected by edge portions having perforations for ease of separation. The practitioner or farmer may thereby simply and reliably remove one dosage in a sachet and make up the formulation as directed for administration to the sick animal. Other suitable packaging will be apparent to those in this industry and no particular limitation is to be inferred. Thus the product may be distributed as a plurality of sachets connected end to end as in a continuous strip pack which may be reeled for storage, or connected edge to edge as sheets of a convenient size, or the discrete sachets may simply be loose filled in a suitable dispenser containing from 10 to 50 sachets.

The invention will now be further described by way of the following examples.

### Example 1

A water soluble powder of the composition shown in the table below is contained in a single 165.9 g sachet which forms an orange solution on mixing with water for oral administration. Apart from the active ingredients listed in the table a drying agent e.g., silicon dioxide is included at up to 3% by weight and about 0.1% by weight of a colouring agent (Sunset Yellow) is included to facilitate product identification after make up. These last ingredients may be varied (i.e., exchanged for functional equivalents) without affecting the efficacy of the composition.

**Table 1a**

| Composition | %w/w |
|---|---|
| **Active Components** | |
| Sodium Citrate | 5.895 |
| Sodium Acetate | 3.279 |
| Sodium Propionate | 1.157 |
| Sodium Chloride | 2.821 |
| Potassium Chloride | 1.796 |
| Dextrose | 81.977 |
| **Auxiliary Additives** | |
| Silicon Dioxide | 3.015 |
| Sunset Yellow (dye) | 0.06 |

The propionate, acetate and citrate ions together yield 93 mmol/l bicarbonate. It is to be noted that the drying agent is insoluble and will remain in the mixing vessel so that on reconstitution with 2 litres of water the available concentrations are as indicated below.

**Table 1b**

| **Component** | millimoles/litre |
|---|---|
| Sodium | 133 |
| Potassium | 20 |
| Chloride | 60 |
| Propionate | 10 |
| Acetate | 33 |
| Citrate | 16.7 |
| Dextrose | 378 |

### Example 2

A water soluble powder of the composition shown in the table below is contained in a single 83.74 g sachet which forms an orange solution on mixing with water for oral administration. Apart from the active ingredients listed in the table a drying agent e.g. silicon dioxide is included at up to 3% by weight and about 0.1 % by weight of a colouring agent (Sunset Yellow) is included to facilitate product identification after make up. These last ingredients may be varied (i.e. exchanged for functional equivalents) without affecting the efficacy of the composition.

**Table 2a**

| **Composition** | % **w/w** |
|---|---|
| **Active components** | |
| Sodium Citrate | 4.681 |
| Sodium Acetate | 3.917 |
| Sodium Propionate | 2.293 |
| Sodium Chloride | 5.589 |
| Potassium Chloride | 3.559 |
| Potassium Dihydrogen Orthophosphate | 1.624 |
| Dextrose | 75.237 |
| **Auxiliary additives** | |
| Silicon dioxide | 3.000 |
| Sunset Yellow (dye) | 0.100 |

The propionate, acetate and citrate ions together yield 50 mmol/l bicarbonate. It is to be noted that the drying agent is insoluble and will remain in the mixing vessel so that on reconstitution with 2 litres of water the available concentrations are as indicated below.

**Table 2b**

| Component | millimoles/litre |
|---|---|
| Sodium | 90 |
| Potassium | 25 |
| Chloride | 60 |
| Phosphate | 5 |
| Propionate | 10 |
| Acetate | 20 |
| Citrate | 6.67 |
| Dextrose | 175 |

The above formulations constitute a single dosage for the treatment of a calf suffering from scour and addresses the areas of electrolyte replacement, fluid replacement, correction of acidosis and provision of an energy source.

The bulk material may be manufactured according to existing pharmaceutical industry practice. Packaging thereof may also be carried out according to usual practice for similar products.

A comparison of the products of this invention with the prior art Life Aid P^{™} and Lectade Plus^{™} is represented in the Table 3 below.

**Table 3**

| | **Life Aid P^{™}** | **Example 1** | **Example 2** | **Lectade Plus^{™}** |
|---|---|---|---|---|
| **Constituent** | mmol/l | mmol/l | mmol/l | mmol/l |
| Glucose | 112.5 | 378.0 | 175.0 | 158.2 |
| Glycine | 41.3 | - | - | 20.0 |
| Citrate | - | 16.7 | 6.7 | 9.5 |
| Propionate | 2.17 | 10.0 | 10.0 | - |
| Acetate | - | 33.0 | 20.0 | - |
| Phosphate | 15.0 | - | 5.0 | 5.0 |
| Sodium | 75.7 | 133.0 | 90.0 | 49.5 |
| Potassium | 15.0 | 20.0 | 25.0 | 20.0 |
| Chloride | 73.6 | 60.0 | 60.0 | 39.3 |
| Osmolarity | 335.3 | 650.7 | 391.7 | 301.5 |

The product of Example 2 was evaluated against Lectade Plus^{™} as a positive control in a model of calf diarrhoea. The model used neonatal calves as the target species and a pathogenic species of *E. Coli* as the test organism (which is widely accepted as being the commonest bacterial cause of diarrhoea in calves. As discussed by Bywater, R. J. (American Journal of Veterinary Research 38, 1983 - 1987, 1977), this model is not purely "coliform"; the *E. Coli* are believed to facilitate the involvement of other pathogenic microorganisms. It can therefore properly be regarded as a "mixed" infection. Moreover, the difficulties inevitably encountered in conducting clinical trials of neonatal diarrhoea and of standardising important aspects of the trial under field conditions make the use of the disease model not only acceptable but preferable to field trials. In addition, the reliable collection of quantitive scientific data under field conditions cannot be undertaken as readily as in a disease model.

Newborn calves of Ayrshire and Friesian breeds (7 Ayrshires and 2 Friesians in each treatment group) were used. Their immunological status was established by the zinc sulphate turbidity test. Each calf was housed in an individual pen and maintained, initially on a milk substitute product (2 I. per day). Clinical measurements and samples for laboratory analyses (see below for details) were taken in healthy calves prior to infection:

E. Coli (strain B44) was used to establish the diarrhoea, following incubation at 34-37°C in Brain Heart Infusion Broth for 16 or 24 hours. Each calf received a 10 ml aliquot of the culture containing 10¹⁰ bacteria per ml orally twice daily for 2 days. Clinical examinations were then carried out twice daily, with particular attention paid to faecal consistency, using the scoring system: 1 normal; 2 semi-solid; 3 diarrhoeic; 4 severely diarrhoeic. Calves with cumulative scores at a given level were deemed to have developed diarrhoea of sufficient severity to enter the treatment phases (Phase II) of the study.

Of the animals proceeding to Phase II, 50% were assigned to Lectade Plus treatment and 50% to LIFE-AID XTRA therapy (formulations of this invention). Each calf received 2 litres of solution twice daily for 2 days, followed for 2 further days by 2 litres of 50:50 mixture of oral re-hydration solution: milk replacer. Animals then received 2 litres of milk replacer alone twice daily.

Efficacy was assessed: (a) by clinical observations and measurements made twice daily and linked to a scoring system; (b) by biochemical analyses on plasma; (c) by measurement of haematological variables and; (d) by estimation of plasma volume using Evans Blue Dye. Variables (b) and (c) were estimated at 24 hour intervals, from the time of commencing fluid therapy up to 96 hours and variables (d) were monitored twice, at 48 hour intervals from the time of commencing fluid therapy.

Prior to treatment, mean faecal scores of 2.89 (LIFE-AID XTRA group) and 2.67 (Lectade Plus group) were obtained, signifying a moderate degree of diarrhoea in both groups. The reduction in faecal score was achieved more rapidly (16 hours) and the magnitude of the response was greater in the Calves receiving LIFE-AID XTRA. Thus the percentage reductions, respectively, at 88 and 96 hours were 46 and 46 (LIFE-AID XTRA group) and 17 and 29 (Lectade Plus group). The overall improvement of animals, as indicated in pre- and post treatment clinical scores was similar in both treatment groups, although the response to LIFE-AID XTRA was again achieved more rapidly. Thus, the pre-treatment score of 1.9 was reduced to 0.3 (16 hours), 0.1 (24 hours) and 0.2 (96 hours) after the commencement of LIFE-AID XTRA treatment. Corresponding values in the Lectade Plus group were 2.3 (pre-treatment), 0.9 (16 hours), 0.6 (24 hours) and 0.2 (96 hours).

In this study a mild degree of acidosis was apparent in both treatment groups at the start of the therapy. The total CO₂ concentration (which may be regarded as a measure of plasma bicarbonate) was 27.0 mmol/l and 28.3 mmol/l at this time in LIFE-AID XTRA and Lectade Plus groups, respectively. This variable was increased only slightly in Lectade Plus treated animals, by 8.6% and 7.2%, at 24 and 48 hours, respectively. Corresponding increases in total CO₂ in the LIFE-AID XTRA treated Calves were 23.9% and 29.0°.6, respectively, at 24 and 48 hours. These differences reflect the greater bicarbonate precursor concentration of LIFE-AID XTRA (almost double that of Lectade Plus).

Compared to the mean pre-infection value, plasma volume was reduced by 6% in the LIFE-AID XTRA group and most of this deficit had been corrected at the 48 hour measuring time. Mean plasma volumes were 3.111 I (pre-infection), 2.81 I (time 0) and 3.05 l (48 hours).

A mild to moderate degree of haemoconcentration, indicated by 9.6% (LIFE-AID XTRA group) and 5.7% (Lectade Plus Group) increases in p.c.v., occurred in both treatment groups following the establishment of infection and prior to the administration of fluids. With LIFE-AID XTRA the efficacy of re-hydration was indicated by a rapid decrease in p.c.v., an effect which was maintained during and following fluid administration. Similar, but somewhat smaller changes were obtained in the Lectade Plus group (Table 4).

**Table 4**

| Mean p.c.v. (n = 9) prior to, during and following oral re-hydration. | | | | |
|---|---|---|---|---|
| | **Life-Aid Xtra** | | **Lectade Plus** | |
| Time | | Change | | Change |
| (hours) | (%) | from control | (%) | from control |
| Pre-infection | 34.2 | - | 36.8 | - |
| 0 | 37.5 | +3.3 | 38.9 | +2.1 |
| 24 | 32.3 | -1.9 | 36.1 | -0.7 |
| 48 | 33.6 | -0.6 | 36.8 | 0.0 |
| 72 | 32.3 | -1.9 | 35.9 | -0.9 |
| 96 | 31.9 | -2.3 | 35.5 | -1.3 |

In summary the comparative trial in scouring calves yielded clinical signs of disease, including scours, and biochemical/haemotological evidence of mild/moderate dehydration and acidosis. In terms both of Clinical responses and biochemical/haematological changes, LIFE-AID XTRA was shown to be at least as effective and, in several important respects, more effective than Lectade Plus. Side-effects were not encountered in either group of Calves. It is therefore concluded that LIFE-AID XTRA will be at least as effective as Lectade Plus in clinical use and hence more effective than the majority of other licensed oral rehydration products.

## Claims

1. A composition to be made up in water at point of use as an oral rehydration formulation, which composition comprises an intimate mixture of an energy source, electrolytes and bicarbonate precursors, which precursors are provided as a plurality of physiologically acceptable carboxylic acid anions with corresponding physiologically acceptable cations including sodium, wherein the bicarbonate precursors are selected from alkali metal salts of a tricarboxylic acid and two other carboxylic acids, the yield of bicarbonate being from about 30 millimole/litre (mmol/l) to about 100 mmol/l of final formulation, and the concentration of sodium being in excess of 80 mmmol/l to about 133 mmol/l of final formulation.

2. An oral rehydration formulation comprising an aqueous solution of an intimate mixture of an energy source, electrolytes, and bicarbonate precursors, which precursors are provided as a plurality of physiologically acceptable carboxylic acid anions with corresponding physiologically acceptable cations including sodium, the yield of bicarbonate being from about 30 millimole/litre (mmol/l) to about 100 mmol/l of final formulation, and the concentration of sodium being in excess of 80 mmol/l to about 133 mmol/l of final formulation.

3. A composition according to claim 1 or claim 2 further comprising potassium chloride and potassium dihydrogen orthophosphate.

4. A composition according to claim 1 wherein the carboxylic acid anions are citrate, acetate and propionate.

5. A composition according to any one of the preceding claims wherein the yield of bicarbonate is from about 50 to about 100 mmol/l of final formulation.

6. A composition according to any one of the preceding claims wherein the amount of energy source is in excess of 160 mmol/l of final formulation.

7. A composition according to claim 6 wherein the energy source is glucose in an amount of from 3 - 7% by weight of the final formulation.

8. A composition according to claim 1 or any one of claims 3 to 7 which is in the form of a substantially dry powder.

9. A composition according to any preceding claim additionally comprising a desiccant.

10. A composition according to any one of claims 1 to 7 which is in the form of a liquid concentrate requiring dilution for administration.

11. A unitary dosage package of components to be made up in water at point of use as an oral rehydration formulation, which composition comprises an intimate mixture (approx. % w/w) of sodium citrate (5.9), sodium acetate (3.3), sodium propionate (1.2), sodium chloride (2.8), potassium chloride (1.8), dextrose (82.0) with the balance consisting of a desiccant and a colouring additive.

12. A veterinary formulation in liquid form for oral administration comprising about 133 millimoles/litre sodium, about 20 millimoles/litre potassium, about 60 millimoles/litre chloride, about 10 millimoles/litre propionate, about 33 millimoles/litre acetate, about 16.7 millimoles/litre citrate, and about 378 millimoles/litre dextrose.

13. A unitary dosage package of components to be made up in water at point of use as an oral rehydration formulation, which composition comprises an intimate mixture (approx. % w/w) of sodium citrate (4.7), sodium acetate (3.9), sodium propionate (2.3), sodium chloride (5.6), potassium chloride (3.6), potassium dihydrogen ortho-phosphate (1.6), dextrose (75.2) with the balance consisting of a desiccant and a colouring additive.

14. A veterinary formulation in liquid form for oral administration comprising about 90 millimoles/litre sodium, about 25 millimoles/litre potassium, about 60 millimoles/litre chloride, about 5 millimoles/litre phosphate, about 10 millimoles/litre propionate, about 20 millimoles/litre acetate, about 7 millimoles/litre citrate, and about 175 millimoles/litre dextrose.

## Patentansprüche

1. Zusammensetzung zum Ansetzen in Wasser bei Gebrauch als orale Formulierung zur Rehydratation, wobei die Zusammensetzung eine innige Mischung aus einem Energiespender, Elektrolyten und Bicarbonat-Vorläufern umfasst, wobei die Vorläufer als eine Vielzahl physiologisch verträglicher Carbonsäureanionen mit entsprechenden physiologisch verträglichen Kationen, einschließlich Natrium, bereitgestellt werden, wobei die Bicarbonat-Vorläufer aus Alkalimetallsalzen einer Tricarbonsäure und zwei anderen Carbonsäuren ausgewählt sind, wobei der Gehalt an Bicarbonat von etwa 30 Millimol/Liter (mmol/l) bis etwa 100 mmol/l der endgültigen Formulierung ist und die Natriumkonzentration größer als 80 mmol/l bis etwa 133 mmol/l der endgültigen Formulierung ist.

2. Orale Formulierung zur Rehydratation, welche eine wässrige Lösung einer innigen Mischung aus einem Energiespender, Elektrolyten und Bicarbonat-Vorläufern umfasst, wobei die Vorläufer als eine Vielzahl physiologisch verträglicher Carbonsäureanionen mit entsprechenden physiologisch verträglichen Kationen, einschließlich Natrium, bereitgestellt werden, wobei der Gehalt an Bicarbonat von etwa 30 Millimol/Liter (mmol/l) bis etwa 100 mmol/l der endgültigen Formulierung ist und die Natriumkonzentration größer als 80 mmol/l bis etwa 133 mmol/l der endgültigen Formulierung ist.

3. Zusammensetzung nach Anspruch 1 oder Anspruch 2, die weiterhin Kaliumchlorid und Kaliumdihydrogenorthophosphat umfasst.

4. Zusammensetzung nach Anspruch 1, wobei die Carbonsäureanionen Citrat, Acetat und Propionat sind.

5. Zusammensetzung nach einem der vorhergehenden Ansprüche, wobei der Gehalt an Bicarbonat von etwa 50 bis etwa 100 mmol/l der endgültigen Formulierung ist.

6. Zusammensetzung nach einem der vorhergehenden Ansprüche, wobei die Menge des Energiespenders größer als 160 mmol/l der endgültigen Formulierung ist.

7. Zusammensetzung nach Anspruch 6, wobei der Energiespender Glucose in einer Menge von 3-7 Gewichts-% der endgültigen Formulierung ist.

8. Zusammensetzung nach Anspruch 1, oder einem der Ansprüche 3 bis 7, die in Form eines im Wesentlichen trockenen Pulvers ist.

9. Zusammensetzung nach einem der vorhergehenden Ansprüche, die zusätzlich ein Trocknungsmittel umfasst.

10. Zusammensetzung nach einem der Ansprüche 1 bis 7, die in Form eines flüssigen Konzentrats ist, das für die Verabreichung verdünnt werden muss.

11. Einheitliche Dosispackung von Bestandteilen, die bei Gebrauch als orale Formulierung zur Rehydratation in Wasser angesetzt wird, wobei die Zusammensetzung eine innige Mischung (etwa % w/w) aus Natriumcitrat (5,9), Natriumacetat (3,3), Natriumpropionat (1,2), Natriumchlorid (2,8), Kaliumchlorid (1,8), Dextrose (82,0) umfasst, wobei der Rest aus einem Trocknungsmittel und einem Farbstoff besteht.

12. Veterinärmedizinische Formulierung in flüssiger Form zur oralen Verabreichung, die etwa 133 Millimol/Liter Natrium, etwa 20 Millimol/Liter Kalium, etwa 60 Millimol/Liter Chlorid, etwa 10 Millimol/Liter Propionat, etwa 33 Millimol/Liter Acetat, etwa 16,7 Millimol/Liter Citrat und etwa 378 Millimol/Liter Dextrose umfasst.

13. Einheitliche Dosispackung von Bestandteilen, die bei Gebrauch als orale Formulierung zur Rehydratation in Wasser angesetzt wird, wobei die Zusammensetzung eine innige Mischung (etwa % w/w) von Natriumcitrat (4,7), Natriumacetat (3,9), Natriumpropionat (2,3), Natriumchlorid (5,6), Kaliumchlorid (3,6), Kaliumdihydrogenorthophosphat (1,6), Dextrose (75,2) umfasst, wobei der Rest aus einem Trocknungsmittel und einem Farbstoff besteht.

14. Veterinärmedizinische Formulierung in flüssiger Form zur oralen Verabreichung, die etwa 90 Millimol/Liter Natrium, etwa 25 Millimol/Liter Kalium, etwa 60 Millimol/Liter Chlorid, etwa 5 Millimol/Liter Phosphat, etwa 10 Millimol/Liter Propionat, etwa 20 Millimol/Liter Acetat, etwa 7 Millimol/Liter Citrat und etwa 175 Millimol/Liter Dextrose umfasst.

## Revendications

1. Composition destinée à être complétée par de l'eau au moment de l'utilisation, sous forme d'une formulation orale de réhydratation, laquelle composition comprend un mélange intime d'une source d'énergie, d'électrolytes et de précurseurs de bicarbonate, lesquels précurseurs sont présentés sous forme d'une pluralité d'anions d'acide carboxylique acceptables d'un point de vue physiologique, avec des cations correspondants, acceptables d'un point de vue physiologique, comprenant le sodium, où les précurseurs de bicarbonate sont choisis parmi les sels de métaux alcalins d'un acide tricarboxylique et deux autres acides carboxyliques, la production du bicarbonate étant comprise entre environ 30 millimoles/libre (mmol/l) et environ 100 mmol/l de formulation finale, et la concentration du sodium étant supérieure à 80 mmol/l et allant jusqu'à environ 133 mmol/l de formulation finale.

2. Formulation orale de réhydratation, comprenant une solution aqueuse d'un mélange intime d'une source d'énergie, d'électrolytes et de précurseurs de bicarbonate, les précurseurs étant mis à disposition sous forme d'une pluralité d'anions acide carboxylique acceptables d'un point de vue physiologique, avec des cations correspondants acceptables d'un point de vue physiologique, comprenant le sodium, la production de bicarbonate étant comprise entre environ 30 millimoles/litre (mmol/l) et environ 100 mmol/l de formulation finale, et la concentration du sodium étant supérieure à 80 mmol/l et allant jusqu'à environ 133 mmol/l de formulation finale.

3. Composition selon la revendication 1 ou la revendication 2, qui comprend en outre du chlorure de potassium et du dihydrogéno-orthophosphate de potassium.

4. Composition selon la revendication 1, dans laquelle les anions d'acide carboxylique sont les anions citrate, acétate et propionate.

5. Composition selon l'une quelconque des revendications précédentes, dans laquelle la production de bicarbonate est comprise entre environ 50 et environ 100 mmol/l de formulation finale.

6. Composition selon l'une quelconque des revendications précédentes, dans laquelle la quantité de la source d'énergie est supérieure à 160 mmol/l de formulation finale.

7. Composition selon la revendication 6, dans laquelle la source d'énergie est le glucose, en une quantité de 3 à 7% en poids, par rapport à la formulation finale.

8. Composition selon la revendication 1 ou l'une quelconque des revendications 3 à 7, qui se présente sous forme d'une poudre essentiellement sèche.

9. Composition selon l'une quelconque des revendications précédentes, qui comprend en outre un déshydratant.

10. Composition selon l'une quelconque des revendications 1 à 7, qui se présente sous forme d'un concentré liquide exigeant une dilution avant administration.

11. Conditionnement posologique unitaire de constituants, destiné à être complété par de l'eau au moment de l'utilisation sous forme d'une formulation orale de réhydratation, laquelle composition comprend un mélange intime (% m/m approximatif) de citrate de sodium (5,9), d'acétate de sodium (3,3), de propionate de sodium (1,2), de chlorure de sodium (2,8), de chlorure de potassium (1,8), de dextrose (82,0), le reste étant constitué d'un déshydratant et d'un additif colorant.

12. Formulation vétérinaire sous forme liquide pour administration orale, comprenant environ 133 millimoles/litre de sodium, environ 20 millimoles/litre de potassium, environ 60 millimoles/litre de chlorure, environ 10 millimoles/litre de propionate, environ 33 millimoles/litre d'acétate, environ 16,7 millimoles/litre de citrate et environ 378 millimoles/litre de dextrose.

13. Conditionnement posologique unitaire de constituants destiné à être complété par de l'eau au moment de l'utilisation en tant que formulation orale de réhydratation, laquelle composition comprend un mélange intime (% m/m approximatif) de citrate de sodium (4,7), d'acétate de sodium (3,9), de propionate de sodium (2,3), de chlorure de sodium (5,6), de chlorure de potassium (3,6), de dihydrogéno-orthophosphate de potassium (1,6), de dextrose (75,2), le reste étant constitué d'un déshydratant et d'un additif colorant.

14. Formulation vétérinaire sous forme liquide pour administration orale, comprenant environ 90 millimoles/litre de sodium, environ 25 millimoles/litre de potassium, environ 60 millimoles/litre de chlorure, environ 5 millimoles/litre de phosphate, environ 10 millimoles/litre de propionate, environ 20 millimoles/litre d'acétate, environ 7 millimoles/litre de citrate et environ 175 millimoles/litre de dextrose.
